# EUROPÄISCHE PATENTSCHRIFT

(11) **EP 0 689 533 B1**
(45) Veröffentlichungstag und Bekanntmachung des Hinweises auf die Patenterteilung: **05.11.1997**
(21) Anmeldenummer: 94910398.0
(22) Anmeldetag: 09.03.1994
(51) Int. Cl.: C07C 219/06, C07C 213/06, C11D 1/62

(54) **VERFAHREN ZUR HERSTELLUNG FARB- UND GERUCHSSTABILER QUATERNIERTER FETTSÄURETRIETHANOLAMINESTER-SALZE**
METHOD OF PREPARING COLOUR- AND ODOUR-STABLE QUATERNARY FATTY-ACID TRIETHANOLAMINE ESTER SALTS
PROCEDE DE PREPARATION DE SELS QUATERNAIRES D'ESTERS DE TRIETHANOLAMINES D'ACIDES GRAS AYANT UNE COULEUR ET UNE ODEUR STABLES

(30) Priorität: 18.03.1993 DE 4308792
(43) Veröffentlichungstag der Anmeldung: 03.01.1996
(73) Patentinhaber: Henkel Kommanditgesellschaft auf Aktien, 40191 Düsseldorf (DE)
(72) Erfinder: PONSATI OBIOLS, Oriol, E-08025 Barcelona (ES); BIGORRA LLOSAS, Joaquim, E-08203 Sabadell (ES)
(86) Internationale Anmeldenummer: EP9400716
(87) Internationale Veröffentlichungsnummer: WO9421596

(56) Entgegenhaltungen:
- EP-A- 0 498 050
- EP-A- 0 525 271
- WO-A-91/01295
- DE-A- 3 815 270

## Beschreibung

Die Erfindung betrifft ein Verfahren zur Herstellung farbund geruchstabiler quaternierter FettsäuretriethanolaminesterSalze, bei dem man Fettsäuren mit Triethanolamin umsetzt, die Veresterungsprodukte mit Peroxidverbindungen und Alkaliboranaten behandelt und die Quaternierung in Gegenwart ausgewählter Stabilisatoren durchführt.

### Stand der Technik

Quaternierte Fettsäuretriethanolaminester-Salze, sogenannte "Esterquats", stellen kationische Tenside dar, die infolge ihrer ausgezeichneten Avivageleistung und ihrer hohen ökotoxiologischen Verträglichkeit zunehmend an Bedeutung gewinnen. Stellvertretend soll hier auf die Veröffentlichungen von O.Ponsati in C.R. **CED-Kongress, Barcelona, 1992, S.167** und R.Puchta in C.R. **CED-Kongress, Sitges, 1993, S.59** verwiesen werden.

Üblicherweise werden die Esterquats in einem zweistufigen Prozeß hergestellt, bei dem man zunächst Triethanolamin mit Fettsäuren - partiell - verestert und das Reaktionsprodukt anschließend alkyliert oder mit Ethylenoxid quaterniert. Als optimaler Katalysator für die Veresterung hat sich unterphosphorige Säure bzw. Natriumhypophosphit bewährt. Im Verlauf der Aufarbeitung, insbesondere bei höheren Temperaturen, kann es jedoch zu einer Zersetzung der unterphosphorigen Säure bzw. deren Salze und zur Bildung kleiner Mengen an Phosphinen kommen, die ihrerseits zu einer geruchlichen Beeinträchtigung des Produktes Anlaß geben. Ein weiteres Problem bei der Herstellung von Esterquats besteht ferner darin, daß die nach Fertigstellung hellfarbigen Produkte nicht unbegrenzt lagerstabil sind und im Laufe der Zeit erheblich nachdunkeln können, was ihre kommerzielle Nutzbarkeit einschränkt.

EP-A-498 050 offenbart Fettsäureester des N-Methyl-N,N,N-trihydroxyethylammonium-methylsulfats und ein Verfahren zu deren Herstellung. In diesem Verfahren wird Triethanolamin mit Fettsäuren verestert, und quaternisiert. Wasserstoffperoxid wird in diesem Verfahren als Bleichmittel verwendet.

Die Aufgabe der Erfindung bestand nun darin, ein Verfahren zur Herstellung von quaternierten Fettsäuretriethanolaminester-Salzen zu entwickeln, das frei von den geschilderten Nachteilen ist.

### Beschreibung der Erfindung

Gegenstand der Erfindung ist ein Verfahren zur Herstellung farb- und geruchstabiler quaternierter Fettsäuretriethanolaminester-Salze der Formel **(I)**, 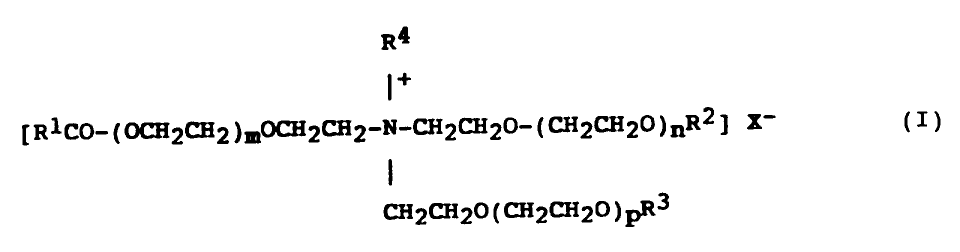 in der
- R¹CO: für einen Acylrest mit 6 bis 22 Kohlenstoffatomen,
- R² und R³: unabhängig voneinander für Wasserstoff oder R¹CO,
- R⁴: für einen Alkylrest mit 1 bis 4 Kohlenstoffatomen oder eine (CH₂CH₂O)_{q}H-Gruppe,
- m, n und p: in Summe für 0 oder Zahlen von 1 bis 12,
- q: für Zahlen von 1 bis 12 und
- X: für Halogenid, Alkylsulfat oder Alkylphosphat
steht, bei dem man
a) Fettsäuren in Gegenwart von unterphosphoriger Säure mit Triethanolamin verestert,
b) die Veresterungsprodukte mit Peroxidverbindungen und Alkaliboranaten versetzt und
c) die Quaternierung in Gegenwart von Phenolderivaten und Hydroxycarbonsäuren als Stabilisatoren durchführt.

Überraschenderweise wurde gefunden, daß erst die Kombination der Merkmale a), b) und c) zu hellfarbigen Esterquats führt, die geruchlich einwandfrei und auch bei längerer Lagerung farbstabil sind. Die Erfindung schließt dabei die Erkenntnis ein, daß von der Vielzahl bekannter Antioxidantien nur ausgewählte Stoffe in der Lage sind, Esterquats gegenüber einer Farbverschlechterung zu stabilisieren. Eine weitere wesentliche Erkenntnis besteht darin, daß auch das vorgeschlagene Stabilisatorsystem seine Aufgabe nur dann zufriedenstellend erfüllen kann, wenn es den Zwischenprodukten - also den noch nicht quaternierten Estern - und nicht den Endprodukten zugesetzt wird.

### Esterquats

Queternierte Fettsäuretriethanolaminester-Salze stellen bekannte Stoffe dar, die man nach den einschlägigen Methoden der präparativen organischen Chemie erhalten kann. In diesem Zusammenhang sei auf die Internationale Patentanmeldung **WO 91/01 295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert.

Vorzugsweise findet das erfindungsgemäße Verfahren Anwendung auf Esterquats, zu deren Herstellung man **Fettsäuren** der Formel **(II)** einsetzt,

**R**^{**1**}**CO-OH (II)**

in der R¹CO die oben angegebene Bedeutung besitzt. Typische Beispiele sind Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt.

Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg-bzw. Palmfettsäure (Iodzahl 0 bis 40) ab.

### Zusatzstoffe

Als **Peroxidverbindungen** kommt neben Percarbonsäuren und Percarbonaten vorzugsweise Wasserstoffperoxid in Betracht. Unter **Alkaliboranaten** sind Lithium-, Kalium- und vorzugsweise Natriumboranat zu verstehen. Vorteilhafterweise werden die Peroxidverbindungen und die Alkaliboranate jeweils in Mengen von 0,005 bis 0,1, vorzugsweise 0,03 bis 0,06 Gew.-% - bezogen auf die Veresterungsprodukte - eingesetzt.

Die **Stabilisatoren** setzen sich aus zwei Komponenten zusammen. Als **Phenolderivate** kommen Bisalkylhydroxytoluole und/oder Bisalkylanisole, insbesondere 2,6-Di-tert.Butyl-4-methyltoluol und 2,6-Di-tert.Butylanisol in Betracht. Als **Hydroxycarbonsäuren** können beispielsweise Citronensäure, Weinsäure und/ oder Ascorbinsäure eingesetzt werden. Vorteilhafterweise werden die Phenolderivate und die Hydroxycarbonsäuren jeweils in Mengen von 100 bis 4000, vorzugsweise 400 bis 900 ppm - bezogen auf den quaternierten Ester - eingesetzt. In einer bevorzugten Ausführungsform des erfindungsgemäßen Verfahrens werden als Stabilisatoren Gemische von 2,6-Di-tert.Butyl-4-methylphenol und Citronensäure eingesetzt.

Die Quaternierung kann in an sich bekannter Weise mit Alkylhalogeniden, (Di-)alkylsulfaten oder Alkylenoxiden - letztere in Gegenwart von (Di-)alkylphosphaten - durchgeführt werden. Vorzugsweise erfolgt die Quaternierung mit Methylchlorid, Dimethylsulfat oder Ethylenoxid.

### Gewerbliche Anwendbarkeit

Die nach dem erfindungsgemäßen Verfahren erhältlichen quaternierten Fettsäuretriethanolaminester-Salze sind hellfarbig, geruchlich einwandfrei und lagerstabil. Sie eignen sich daher zur Herstellung von Wasch-, Spül-, Reinigungs- und Avivagemitteln sowie Produkten zur Haar- und Körperpflege, in denen sie in Mengen von 1 bis 50, vorzugsweise 5 bis 35 Gew.-% - bezogen auf die Mittel - enthalten sein können.

Die folgenden Beispiele sollen den Gegenstand der Erfindung näher erläutern, ohne ihn darauf einzuschränken.

### Beispiele

### Allgemeine Herstellvorschrift für Esterquats:

a) **Veresterung**. In einem 1-l-Dreihalskolben mit Rührer, Innenthermometer und Destillationsaufsatz wurden 324 g (1,2 mol) teilgehärtete C_{16/18}-Palmfettsäure (Iodzahl = 40), 149 g (1 mol) Triethanolamin und 0,5 g 50 gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Talgfettsäuretriethanolaminester abgekühlt und der Reaktionsansatz entspannt. Der Ester wurde zunächst bei 60°C mit Wasserstoffperoxid und anschließend mit Natriumboranat versetzt. Nach jeder Zugabe wurde die Mischung 30 min gerührt. Danach wurden die Stabilisierungsmittel zugesetzt.
b) **Quaternierung**. In einem 1-l-Dreihalskolben mit Rührer, Tropftrichter und Rückflußkühler wurde eine Mischung von 450 g (1 mol) des Esters aus b) in 200 ml Isopropylalkohol vorgelegt und unter Rühren auf 45°C erhitzt. Innerhalb von 2 h wurden 119 g (0,95 mol) Dimethylsulfat zugetropft. Nach Beendigung der Zugabe wurde die Mischung weitere 2 h bei 60°C gerührt und nichtumgesetztes DMS durch Zusatz von 4 g (0,05 mol) Glycin zerstört. Anschließend wurde das Lösungsmittel im Wasserstrahlvakuum abgetrennt. Die Ausbeute an Esterquat betrug 95 % der Theorie. Einzelheiten zu den Versuchsansätzen und den Ergebnissen sind Tab.1 zu entnehmen:

**Tab.1:**

| Stabilisierung von Esterquats Prozentangaben als Gew.-% | | | | | | | |
|---|---|---|---|---|---|---|---|
| Bsp. | c(WP) % | c(NB) % | S1 | S2 | c(S1) ppm | c(S2) ppm | Farbe Klett |
| 1 | 0,05 | 0,01 | BHT | CA | 900 | 400 | 50 |
| 2 | 0,05 | 0,01 | DBA | CA | 900 | 400 | 75 |
| 3 | 0,05 | 0,01 | BHT | AA | 900 | 500 | 75 |
| V1 | - | 0,01 | BHT | CA | 900 | 400 | 200 |
| V2 | 0,05 | - | BHT | CA | 900 | 400 | 150 |
| V3 | 0,05 | 0,01 | - | - | - | - | 200 |
| V4 | 0,05 | 0,01 | BHT | - | 900 | - | 150 |
| V5 | 0,05 | 0,01 | - | CA | - | 400 | 150 |
| V6 | 0,05 | 0,01 | BHT | CA | 900 | 400 | 100* |
| V7 | 0,05 | 0,01 | BHT | TP | 900 | 400 | 150 |

| | | | | | | | |
|---|---|---|---|---|---|---|---|
| Legende: c(WP) = Konzentration Wasserstoffperoxid c(NB) = Konzentration Natriumboranat c(S) = Konzentration Stabilisator Farbe = Gemessen in einem Klett-Photometer, 5 % Aktivsubstanz, 1 cm-Rundküvette nach 30 d Lagerung (20°C) BHT = 2,6-Di-tert.Butyl-4-methylphenol DBA = 2,6-Di-tert.Butylanisol CA = Citronensäure AA = Ascorbinsäure TP = Tocopherol * = Stabilisator nach der Quaternierung zugesetzt | | | | | | | |

## Patentansprüche

1. Verfahren zur Herstellung farb- und geruchstabiler quaternierter Fettsäuretriethanolaminester-Salze der Formel **(I)**, 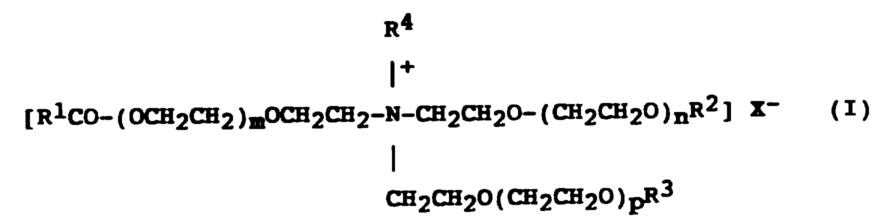 in der
R¹CO für einen Acylrest mit 6 bis 22 Kohlenstoffatomen,
R² und R³ unabhängig voneinander für Wasserstoff oder R¹CO,
R⁴ für einen Alkylrest mit 1 bis 4 Kohlenstoffatomen oder eine (CH₂CH₂O)_{q}H-Gruppe,
m, n und p in Summe für 0 oder Zahlen von 1 bis 12,
q für Zahlen von 1 bis 12 und
X für Halogenid, Alkylsulfat oder Alkylphosphat
steht, bei dem man
a) Fettsäuren in Gegenwart von unterphosphoriger Säure mit Triethanolamin verestert,
b) die Veresterungsprodukte mit Peroxidverbindungen und Alkaliboranaten versetzt und
c) die Quaternierung in Gegenwart von Phenolderivaten und Hydroxycarbonsäuren als Stabilisatoren durchführt.

2. Verfahren nach Anspruch 1, **dadurch gekennzeichnet**, daß man Fettsäuren der Formel **(II)** einsetzt,
**R**^{**1**}**CO-OH (II)**
einsetzt, in der R¹CO die oben angegebene Bedeutung besitzt.

3. Verfahren nach Anspruch 1, **dadurch gekennzeichnet**, daß man die Fettsäuren und das Triethanolamin im molaren Verhältnis von 1,1 : 1 bis 3,0 : 1 einsetzt.

4. Verfahren nach Anspruch 1, **dadurch gekennzeichnet**, daß man als Peroxidverbindungen Wasserstoffperoxid einsetzt.

5. Verfahren nach Anspruch 1, **dadurch gekennzeichnet**, daß man als Alkaliboranat Natriumboranat einsetzt.

6. Verfahren nach Anspruch 1, **dadurch gekennzeichnet**, daß man die Peroxidverbindungen und die Alkaliboranate jeweils in Mengen von 0,005 bis 0,1 Gew.-% - bezogen auf die Veresterungsprodukte - einsetzt.

7. Verfahren nach Anspruch 1, **dadurch gekennzeichnet**, daß man als Phenolderivate Bisalkylhydroxytoluole und/oder Bisalkylanisole einsetzt.

8. Verfahren nach Anspruch 1, **dadurch gekennzeichnet**, daß man als Hydroxycarbonsäuren Citronensäure, Weinsäure und/oder Ascorbinsäure einsetzt.

9. Verfahren nach Anspruch 1, **dadurch gekennzeichnet**, daß man die Phenolderivate und die Hydroxycarbonsäuren jeweils in Mengen von 100 bis 4000 ppm - bezogen auf den quaternierten Ester - einsetzt.

10. Verfahren nach Anspruch 1, **dadurch gekennzeichnet**, daß man als Stabilisatoren Gemische von 2,6-Di-tert.Butyl-4-methylphenol und Citronensäure einsetzt.

11. Verfahren nach Anspruch 1, **dadurch gekennzeichnet**, daß man die Quaternierung mit Methylchlorid oder Dimethylsulfat durchführt.

12. Verfahren nach Anspruch 1, **dadurch gekennzeichnet**, daß man die Quaternierung mit Ethylenoxid durchführt.

## Claims

1. A process for the production of color- and odor-stable quaternized fatty acid triethanolamine ester salts corresponding to formula **(I)**: 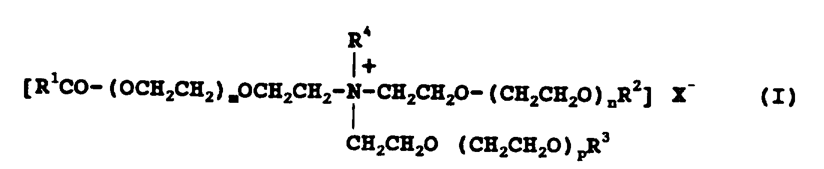 in which
R¹CO is an acyl radical containing 6 to 22 carbon atoms,
R² and R³ independently of one another represent hydrogen or have the same meaning as R¹CO,
R⁴ is an alkyl radical containing 1 to 4 carbon atoms or a (CH₂CH₂O)_{q}H group,
m, n and p together = 0 or a number of 1 to 12 and
X is a halide, alkyl sulfate or alkyl phosphate,
in which
a) fatty acids are esterified with triethanolamine in the presence of hypophosphorous acid,
b) peroxide compounds and alkali metal boranates are added to the esterification products and
c) the quaternization is carried out in the presence of phenol derivatives and hydroxycarboxylic acids as stabilizers.

2. A process as claimed in claim 1, **characterized in that** fatty acids corresponding to formula **(II)**
R¹CO-OH (II)
in which R¹CO is as defined above, are used.

3. A process as claimed in claim 1, **characterized in that** the fatty acids and the triethanolamine are used in a molar ratio of 1.1:1 to 3.0:1.

4. A process as claimed in claim 1, **characterized in that** hydrogen peroxide is used as the peroxide compound.

5. A process as claimed in claim 1, **characterized in that** sodium boranate is used as the alkali metal boranate.

6. A process as claimed in claim 1, **characterized in that** in that the peroxide compounds and the alkali metal boranates are used in quantities of 0.005 to 0.1% by weight, based on the esterification products.

7. A process as claimed in claim 1, **characterized in that** bis-alkyl hydroxytoluenes and/or bis-alkyl anisoles are used as the phenol derivatives.

8. A process as claimed in claim 1, **characterized in that** citric acid, tartaric acid and/or ascorbic acid is/are used as the hydroxycarboxylic acids.

9. A process as claimed in claim 1, **characterized in that** the phenol derivatives and the hydroxycarboxylic acids are each used in quantities of 100 to 4000 ppm, based on the quaternized ester.

10. A process as claimed in claim 1, **characterized in that** mixtures of 2,6-ditert. butyl-4-methyl phenol and citric acid are used as stabilizers.

11. A process as claimed in claim 1, **characterized in that** the quaternization is carried out with methyl chloride or dimethyl sulfate.

12. A process as claimed in claim 1, **characterized in that** the quaternization is carried out with ethylene oxide.

## Revendications

1. Procédé de préparation de sels d'esters de triéthanolamine et d'acides gras quaternisés de formule I, stables quant à la couleur et à l'odeur, 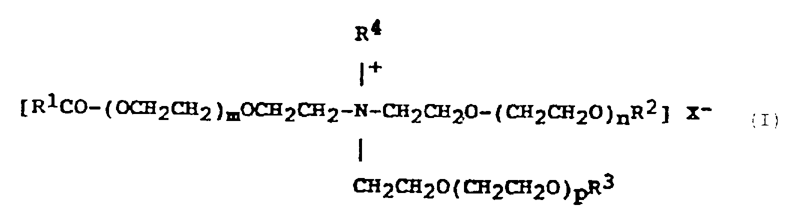 dans laquelle,
R¹CO représente un radical acyle ayant 6 à 22 atomes de carbone,
R² et R³ indépendamment l'un de l'autre, représentent de l'hydrogène ou R¹CO,
R⁴ représente un radical alkyle ayant 1 à 4 atomes de carbone ou un groupe (CH₂CH₂O)_{q}H-
m, n et p au total représentent O ou des nombres allant de 1 à 12,
q représente des nombres de 1 à 12 et
X représente un halogènure, un alkylsulfate ou un phosphate d'alkyle
dans lequel,
a) on estérifie les acides gras en présence d'acide hypophosphoreux avec la triéthanolamine,
b) on additionne les produits d'estérification avec des composés peroxydiques et des boranates de métal alcalin et,
c) on effectue la quaternisation en présence de dérivés de phénol et d'acides hydroxycarboxyliques comme agents stabilisants.

2. Procédé selon la revendication 1,
caractérisé en ce qu' on met en oeuvre des acides gras de formule (II)
R¹CO-OH (II)
dans laquelle R¹CO possède la signification fournie ci-dessus.

3. Procédé selon la revendication 1,
caractérisé en ce qu'
on met en oeuvre les acides gras et la triéthanolamine dans un rapport molaire allant de 1,1:1 à 3,0:1.

4. Procédé selon la revendication 1,
caractérisé en ce qu'
on met en oeuvre comme dérivé peroxydique du peroxyde d'hydrogène.

5. Procédé selon la revendication 1,
caractérisé en ce qu'
on met en oeuvre comme boranate de métal alcalin, le boranate de sodium.

6. Procédé selon la revendication 1,
caractérisé en ce qu'
on met en oeuvre les composés peroxydiques et les boranates de métal alcalin, respectivement en quantités allant de 0,005 à 0,1 % en poids rapporté aux produits d'estérification.

7. Procédé selon la revendication 1,
caractérisé en ce qu'
on met en oeuvre comme dérivés phénoliques des bis-alkylhydroxytoluènes et/ou des bis-alkylanisols.

8. Procédé selon la revendication 1,
caractérisé en ce qu'
on met en oeuvre comme acides hydroxycarboxyliques de l'acide citrique, de l'acide tartrique et/ou de l'acide ascorbique.

9. Procédé selon la revendication 1,
caractérisé en ce qu'
on met en oeuvre les dérivés phénoliques et les acides hydroxycarboxyliques respectivement en quantité allant de 100 à 4000 ppm, rapporté aux esters quaternisés.

10. Procédé selon la revendication 1,
caractérisé en ce qu'
on met en oeuvre comme agents stabilisants des mélanges de 2,6-diterbutyl-4-méthylphénol et d'acide citrique.

11. Procédé selon la revendication 1,
caractérisé en ce qu'
on effectue la quaternisation avec du chlorure de méthyle ou du sulfate de diméthyle.

12. Procédé selon la revendication 1,
caractérisé en ce qu'
on effectue la quaternisation avec de l'oxyde d'éthylène.
